# EUROPEAN PATENT SPECIFICATION

(11) **EP 2 253 228 B1**
(45) Date of publication and mention of the grant of the patent: **22.03.2017**
(21) Application number: 10162445.0
(22) Date of filing: 10.05.2010
(51) Int. Cl.: A23L 29/00, A23L 33/15, A23L 33/00, A61K 31/047, A61K 31/4045, A61K 31/519, A23L 33/10

(54) **COMPOSITION FOR CONTROLLING AND IMPROVING FEMALE AND MALE GAMETOGENESIS**
Zusammensetzung zur Steuerung und Verbesserung der weiblichen und männlichen Gametogenese
Composition pour contrôler et améliorer la gamétogenèse mâle et femelle

(30) Priority: 19.05.2009 IT MI20090875
(43) Date of publication of application: 24.11.2010
(73) Proprietor: LO. LI. Pharma S.r.l., 00132 Roma (IT)
(72) Inventor: Unfer, Vittorio, 00155 Roma (IT)
(74) Representative: Gervasi, Gemma

(56) References cited:
- WO-A2-2008/155651
- US-A1- 2002 182 196
- US-A1- 2009 017 143
- TAMURA H ET AL: "Melatonin and the ovary: physiological and pathophysiological implications", FERTILITY AND STERILITY, ELSEVIER SCIENCE INC, NEW YORK, NY, USA, vol. 92, no. 1, 1 September 2008 (2008-09-01), pages 328-343, XP026542756, ISSN: 0015-0282 [retrieved on 2008-09-18]
- BELLIPANNI, G.; DI MARZO, F.; BLASI, F.; DI MARZ, A.: "Effects of melatonin in perimenopausal and menopausal women", ANN. N.Y. ACAD. SCI., vol. 1057, 1 January 2005 (2005-01-01), pages 393-402, XP002566456,

## Description

The present invention relates to a composition for controlling and improving female and male gametogenesis that is adapted to improve all the natural processes of folliculogenesis, oocyte maturation, ovulation and luteal phase (all described as spermatogenesis in the male) (i) in women affected by ovulation disorders, such as for example polycystic ovary syndrome or genito-pelvic endometriosis, or (ii) in women in whom this physiological process is less efficient, for example due to biological age, or (iii) in women subjected to medically assisted procreation (MAP) techniques wherein hormone treatment with gonadotropins negatively alters these processes.

Folliculogenesis is a natural process adapted to cause the growth, maturation and expulsion of a mature oocyte on the part of the follicle, a fundamental moment of the fertility of women, and the subsequent transformation of the follicle into a corpus luteum, with consequent production of progesterone, a hormone that is decisive in implantation and in maintaining pregnancy in the initial phases.

All these processes are regulated by hormones (mainly FSH and LH, but also by anti-Müllerian hormone, Inhibin A, Inhibin B, and others) and by paracrine activity molecules that act as local/tissue messengers.

FSH and LH, which are produced at the hypothalamic-hypophyseal level, at the ovarian level, cause development of the follicle and of the events described above at very precise times and in very precise manners.

FSH and LH are produced daily in different concentrations, such as to induce very specific phases of the ovulation cycle (follicular phase: ↑ FSH ↓ LH; ovulatory phase: ↑ FSH ↑ LH; luteal phase: ↓ FSH ↑ LH).

Their action depends on a large number of molecules (second messengers) that transmit messages inside the target cells of FSH and LH.

The same hormones and mechanisms of action are then involved in a similar manner at the testicular level, control spermatogenesis and cause production of mature spermatozoa.

Folliculogenesis is altered in some pathologies of the reproductive age, such as (i) ovarian micropolycystosis, a medical condition characterized by irregular menstrual cycles, lack of ovulation and excess of male traits in women as a consequence of an altered ovarian production of androgens, which is the effect of an altered FSH/LH ratio; or (ii) genito-pelvic endometriosis, characterized by reduced ovarian sensitivity to FSH/LH hormones, as a consequence of a chronic irritation caused by the presence of endometrial tissue within the ovarian parenchyma.

Folliculogenesis becomes less efficient (in terms of production of oocytes of good quality, i.e., "mature" ones, and therefore of pregnancies) as a woman gets older, because the ovarian cells that take part in this process (theca and granulosa cells), due to physiological aging and accumulation of free radicals, reduce the activity of these maturing processes for which the first signal derives from the activity of FSH and LH.

Moreover, folliculogenesis is impaired pharmacologically in women subjected to hormone treatments for MAP, regardless of the indications for these treatments.

Exogenous administration of high doses of FSH and LH (in the various pharmacological formulations: Gonal-F, Puregon, Fostimon) or hMG (Meropur) alters the physiological oocyte maturation process with respect to a natural cycle.

In in vitro fertilizations (IVF-ET), the purpose is to recover a large number of oocytes and high dosages of gonadotropins are necessary in order to do so.

The therapeutic efficiency of procedures for in vitro fertilization (IVF-ET) is linked tightly to the number of zygotes obtained, which in turn depends on the number of harvested oocytes. Accordingly, ovarian hyperstimulation is indispensable for increasing the number of oocytes, which is one in the natural menstrual cycle. Various protocols and active ingredients have been used so far (clomiphene citrate, hMG, purified FSH, recombinant FSH, agonists or antagonists of GnRH) with the goal of obtaining an optimum number of oocytes.

Pharmacological ovarian hyper-stimulation provides for the combination of various pharmaceutical drugs that are able to achieve stimulation of the ovaries and the production of many oocytes. Moreover, an analogue of GnRH (Decapeptyl®) is often used which suppresses the synthesis of the gonadotropic hypophyseal hormones (FSH, LH) and induces a blockage of the natural cycle.

The final step of the stimulation consists in inducing ovulation. The oocyte can be fertilized with the spermatozoon only if it has resumed its meiotic division and has expelled the first polar body. It is then considered mature. In a natural cycle, oocyte maturation and ovulation are induced by a peak in LH, the luteinizing hormone. In a stimulated cycle, the administration of hCG (pregnancy hormone) makes it possible to mimic the action of LH. One then speaks of ovulation induction. Several natural or synthetic hormones, such as urinary hCG, recombinant hCG or recombinant LH, can be used for this purpose.

At this point, pharmacologically induced ovulation can be used to increase the likelihood of conception by way of techniques for medically assisted procreation of the first level (intrauterine inseminations) or of the second level (in vitro fertilizations).

When a low sperm count makes classic fertilization impossible, one resorts to ICSI, i.e., microinjection of the single spermatozoon into the mature oocyte.

In the male, spermatogenesis is less efficient (in terms of production of motile and morphologically regular sperm) as age increases, because the testicular cells involved in this process (Sertoli cells and Leydig cells), due to physiological aging and to the accumulation of free radicals, reduce the activity of the maturation processes whose first signal derives from the activity of FSH and LH.

In men as well as in women, hypophyseal gonadotropins play a fundamental role in spermatogenesis. It is possible to obtain adequate spermatogenesis from their correct ratio and from the possibility of achieving a proper testicular response to gonadotropin stimulus.

In US 2002/182196 it is described a supplement composition for normalising impaired or deteriorating neurological functions in humans that can contain inositol and folic acid and melatonin.

WO 2008/155651 describes a composition which is capable of controlling the induction of ovulation in a subject, particularly if the subject is affected by PCOS, hyperinsulinemia or both diseases.

Tamura et al. "Melatonin and the ovary:physiological and pathophysiological implications "FERTILITY AND STERILITY, ELSEVIER SCIENCE INC, NEW YORK, NY, USA vol. 92, no. 1, September 2008 (2008-09-019 PAGES 328 - 343, reports melatonin's essential functions in the human ovarycapable of improving ovarian function and oocyte quality.

Bellipanni et al. "Effects of melatonin in perimenopausal and menopausal women "ANN. N.Y. ACAD. SCI., vol. 1057, 1 january 2005 (2005-01-01) pages 393 - 402 describes the effects of nocturnal melatonin administration in perimenopausal and menopausal women.

Therefore, for women there is the need to provide a composition that is able to control and improve normal ovulatory activity in various situations, both physiological and pathological ones, and in order to antagonize the negative effects caused by the use of hormones for increasing fertility.

Moreover, for men there is the need to provide a composition that can restore or improve the defects of spermatogenesis due to FSH-LH-dependent hormone imbalances.

The aim of the present invention is to provide a composition that is able to improve folliculogenesis in all its stages or to restore it where it is absent or insufficient.

Another object of the invention is to support for its entire duration (about 28 days) the physiological ovulatory cycle of women in all its phases(follicular, FSH dependent; ovulatory, FSH/LH dependent; luteal, LH dependent).

Still another object of the invention is to improve the ovulatory process in women subjected to hormone treatments with high dosages of FSH and LH for medically assisted procreation techniques.

This aim and these and other objects are achieved by a composition consisting of 1000 to 4000 parts by weight of myoinositol and 1 to 5 parts by weight of melatonin, and optionally from 0.2 to 0.8 parts by weight, of folic acid.

In one aspect, the present invention provides a composition consisting of 1000 to 4000 parts by weight of myoinositol and 1 to 5 parts by weight of melatonin and optionally from 0.2 to 0.8 parts by weight of folic acid for use as a food supplement or as a pharmaceutical drug.

In another aspect, the present invention provides a composition consisting of 1000 to 4000 parts by weight of myoinositol and from 1 to 5 parts by weight of melatonin and optionally from 0.2 to 0.8 parts by weight of folic acid for use as a pharmaceutical drug for administration to a female or male subject.

In a further aspect, the present invention provides a unit form of dosage of the composition that comprises from 1 to 4 g of myoinositol, from 1 to 5 mg of melatonin and optionally from 200 to 800 µg of folic acid.

In a further aspect, the present invention provides for the use of a composition that consists of 1000 to 4000 parts by weight of myoinositol and from 1 to 5 parts by weight of melatonin and optionally from 0.2 to 0.8 parts by weight of folic acid for the preparation of a pharmaceutical drug for the treatment of micropolycystic ovary, genito-pelvic endometriosis, ovulation disorders.

The composition according to the present invention may also comprise only myoinositol, melatonin and optionally folic acid, in the same proportions as indicated in the present description.

Other characteristics of the invention will be presented in the following detailed description.

Myoinositol belongs to the group of so-called non-B vitamins and constitutes the main isoform of inositol.

It is known to use this compound in the treatment of constipation, baldness and for lowering the cholesterol level in the blood, becoming thus an effective factor for protection against cardiovascular diseases.

It is known, moreover, that the combined use of inositol and choline also has a positive effect on peripheral diabetic neuropathies, on hypoglycemic subjects and in protecting the liver, kidneys and heart.

The usefulness of inositol for nutrition of brain cells and its necessary role in the growth and survival of cells of bone marrow, ocular membranes and bowels has also been observed.

Finally, the sedative action of inositol has been demonstrated; this compound not only acts as a tranquilizer but also has no side effects. For this reason, inositol is used to cure subjects affected by insomnia, schizophrenia or with a high level of copper and a low level of zinc in their serum.

Melatonin (N-acetyl-5-methoxytryptamine) is an indolamine, originally found in the hypophysis, which derives from the biosynthesis of an essential amino acid, tryptophan, by way of the production of intermediate compounds derived from serotonin.

It is known that melatonin is involved in the mechanism that regulates body rhythms and keeps them synchronized with those of the external environment, exhibits activity for stimulation of the immune system, has rebalancing effects on cardiovascular function, on bone remodeling, on body mass regulation and on the reproductive system.

Moreover, it is known that melatonin has a broad antioxidant action, preventing damage caused by an excess of free radicals and suggesting its use in the treatment of various disorders, including tumors, immune disorders, Alzheimer's disease, diabetes and viral infections.

Folic acid (folate) is vitamin B9, a substance which is not produced by the body and therefore must be obtained from food or by way of the action of the intestinal flora.

In recent decades, folic acid has been recognized as essential in the prevention of neonatal malformations; moreover, it has been demonstrated that a lack of folic acid in the first phases of pregnancy increases greatly the risk of malformation of the fetus and can lead more easily to negative outcomes, such as for example intrauterine growth retardation, premature delivery or placental lesions.

Folic acid contributes to a lowering of the levels of the amino acid homocysteine, which is associated with the risk of cardiovascular diseases and heart attacks. Moreover, folic acid appears to play a role, which has not yet been clarified, in the prevention of other defects and congenital malformations, such as labiopalatoschisis and some congenital cardiac defects.

The present inventor has found that the association of these compounds (myoinositol - melatonin - optionally folic acid), when administered in well-defined daily dosages, is such as to act synergistically, to the point of improving considerably FSH- and LH-dependent actions at the ovary level, be they endogenous or exogenous.

The inventor of the present invention has found, moreover, that said composition is adapted to implement and improve the action of FSH and LH gonadotropins during the entire phase of the ovarian cycle of a woman, and is highly reliable, relatively easy to provide and has competitive costs.

The inventor of the present invention has found, in fact, that the simultaneous administration of myoinositol and melatonin makes it possible to provide the physiological substrate that implements and improves qualitatively the response of ovarian cells to FSH and LH.

Moreover, the simultaneous administration of the two compounds ensures the constant presence of both intrafollicular and intracellular second messengers, particularly myoinositol for FSH and melatonin for LH, which ensure the formation of a mature oocyte, i.e., an oocyte which is functional and of good quality, which is an index of good fertility for a woman.

Myoinositol, in a quantity of at least 1 g/day, can promote follicle growth by amplifying the hypophyseal signal of FSH.

Melatonin, in a quantity of at least 1 mg/day, can facilitate, in the first days of the cycle, the recruitment of the dominant follicle (FSH-LH-mediated message) and then in mid-cycle it can promote the LH peak (i.e., the ovulation).

Since the ovulatory cycle is a continuous balance of FSH- and LH-mediated stimuli, the simultaneous administration of the two compounds is useful also after ovulation in order to maintain or support the luteal phase, which is essential for implantation and maintenance of a pregnancy.

Folic acid, in a quantity of at least 200 µg/day, has proved effective in controlling the tendency to hypercoagulation that is present mainly in patients subjected to ovulation induction.

Its presence at this dosage is already useful to prevent neural tube defects in patients who desire to have children.

The composition of the present invention allows the administration in a single formulation of all the active ingredients of the composition mentioned above.

The inventor has found, moreover, that folic acid, administered simultaneously with the active ingredients that constitute the product, by way of a synergistic action, augments its benefits mainly in terms of ovarian maturity.

The simultaneous presence of myoinositol - melatonin - folic acid in a single formulation enhances the individual properties of the three substances, constituting a preparation that can restore optimal folliculogenesis, guarantee the growth and development of a mature oocyte, support the activity of the corpus luteum and thus promote pregnancy in women of reproductive age.

In a preferred embodiment, the composition of the present invention comprises myoinositol, melatonin and folic acid, if present, in a ratio expressed in parts by weight of 1500-2500 : 2 - 4 : 0.3 - 0.5; in an even more preferred manner, said ratio, expressed in parts by weight, is 2000 : 3 : 0.4.

In a further aspect, the present invention provides a unit dosage form of the composition according to the present invention which comprises or is constituted by 1 to 4 g of myoinositol, 1 to 5 mg of melatonin and 200 to 800 µg of folic acid.

Preferably, said unit dosage form of the composition comprises, or is constituted by, 2 g or more of myoinositol, 3 mg or more of melatonin, and 400 µg or more of folic acid.

A preferred example of dosage form comprises 2 g of myoinositol, 3 mg of melatonin and 400 µg of folic acid.

Another aspect of the present invention relates to the use of the described composition for preparing a pharmaceutical drug for treating micropolycystic ovary, genito-pelvic endometriosis and ovulation disorders.

In a preferred embodiment, said administration is in a daily quantity that comprises 1 to 4 g of myoinositol, 1 to 5 mg of melatonin and, if folic acid is present, 200 to 800 µg of folic acid.

Preferably, said administration is in a daily quantity that comprises 1.5 to 2.5 g of myoinositol, 2 to 4 mg of melatonin and, if folic acid is present, 300 to 500 µg of folic acid.

Even more preferably, said administration is in a daily quantity that comprises 2 g or more of myoinositol, 3 mg or more of melatonin and, if folic acid is present, 400 µg or more of folic acid.

The subject to whom the composition according to the present invention is administered can be a woman with menstrual cycle disorders, a woman affected by micropolycystic ovary syndrome, in which FSH and LH production are altered, or a woman affected by genito-pelvic endometriosis.

The composition according to the present invention
is in particular indicated for all cases of alteration of the rhythm and duration of the cycle (secondary amenorrhea, oligomenorrhea, polymenorrhea, etcetera). For this reason the composition according to the present invention is further indicated for juvenile and
climacteric dysfunctional menometrorrhagias (i.e., ovulation disorders). Indeed, in one embodiment, the subject to whom said composition is administered is a woman affected by adolescent-age ovulation disorders, or a woman affected by perimenopausal-age ovulation disorders.

Moreover, the present composition can be used by all women affected by fertility reduction due to advanced biological age (>35 years) who wish to become pregnant. In these subjects, the reproductive system is rendered less efficient by an excess of free radicals (tissue aging) and by a hormone stimulus (FSH and LH) that is not always effective.

In one embodiment, the subject to whom the composition described herein is administered is a woman affected by fertility reduction due to a reduced ovarian reserve (FSH > 10 mUI/ml); in fact, administration of the product may advantageously implement an insufficient follicular and ovarian activity.

The composition is then indicated in all cases in which women, even
if they are fertile, are subjected to induction of ovulation with exogenous gonadotropins (FSH and/or LH) for MAP, because it provides better control of the ovarian response and a reduction in the incidence of hyperstimulation syndrome.

This mechanism of action is useful in patients who are subjected to cycles of induction of ovulation, because it can reduce the quantity of pharmaceutical drug (FSH and/or LH) used, reducing negative effects.

In other words, the subject to whom the composition according to the present invention is administered can be a woman subjected to induction of ovulation for medically assisted procreation.

In one embodiment, the subject to whom the composition according to the present invention is administered is affected by spermatogenesis disorders..

Tables 1 - 6 present experimental results of the treatment with myoinositol and melatonin in patients for whom this product is intended.

**TABLE 1. Effect of six months of daily treatment with the myoinositol - melatonin combination on menstrual disorders of puberty**

| | Myoinositol + melatonin | | Placebo | |
|---|---|---|---|---|
| | N=12 | | N=12 | |
| | Before treatment | After treatment | Before treatment | After treatment |
| Average duration of menstrual cycle (days) | 38 ± 12 | 31 ± 5 | 37 ± 13 | 36 ± 11 |
| No. of ovulation cycles | | 4.5 ± 0.8 | | 1.2 ± 0.7 |
| Serum progesterone (ng/ml) | | 10.4 ± 3.2 | | 3.1 ± 2.0 |

**TABLE 2. Effect of six months of daily treatment with the myoinositol - melatonin combination on perimenopausal-age menstrual disorders**

| | Myoinositol + melatonin | | Placebo | |
|---|---|---|---|---|
| | N=12 | | N=13 | |
| | Before treatment | After treatment | Before treatment | After treatment |
| Average duration of menstrual cycle (days) | 21 ± 3 | 25 ± 4 | 22 ± 3 | 21 ± 3 |
| No. of menometrorrhagic cycles (> 6days) | 5 ± 2 | 2 ± 2 | 5 ± 2 | 5 ± 2 |
| Serum hemoglobin (g/dl) | 11 ± 3 | 12 ± 3 | 11 ± 3 | 9 ± 2 |

**TABLE 3. Effect of six months of daily treatment with the myoinositol - melatonin combination on menstrual disorders of a patient with micropolycystic ovary.**

| | Myoinositol + melatonin | |
|---|---|---|
| | N=10 | |
| | Before treatment | After treatment |
| Average length of menstrual cycle (days) | 37 ± 9 | 30 ± 5 |
| FSH | 4.5 ± 2.1 | 3.8 ± 1.9 |
| LH | 7.6 ± 3.1 | 4.2 ± 1.9 |
| LH / FSH | 1.7 | 1.1 |
| Progesterone (ng/ml) | 2.4 ± 0.9 | 11.3 ± 3.8 |
| No. of ovulatory cycles / 6 months | | 3.2 |
| % patients with regular cycles | | 6/10 |

**TABLE 4. Effect of three months of daily treatment with the myoinositol - melatonin combination on the outcome of the induction of ovulation with gonadotropins in patients with high FSH (> 10 mUI/ml)**

| | Myoinositol + melatonin | Placebo |
|---|---|---|
| | N=8 | N=8 |
| Average no. of oocytes recovered at pick-up | 5 ± 2 | 3 ± 1 |
| Average no. of mature oocytes (M II) recovered at pick-up | 4 ± 1 | 1 ± 1 |
| Average no. of transferred embryos | 2 | 1 |
| % pregnancy | 2/8 | 0/8 |

**TABLE 5. Effect of three months of daily treatment with the myoinositol - melatonin combination on the outcome of the induction of ovulation with gonadotropins in patients with age > 35 yrs**

| | Myoinositol + melatonin | Placebo |
|---|---|---|
| | N=10 | N=10 |
| Average no. of oocytes recovered at pick-up | 8 ± 2 | 8 ± 2 |
| Average no. of mature oocytes (M II) recovered at pick-up | 7 ± 2 | 6 ± 3 |
| Average no. of transferred embryos | 2 | 1 |
| % pregnancy | 4/10 | 2/10 |

**TABLE 6. Effect of three months of daily treatment with the myoinositol - melatonin combination on parameters of the seminal fluid in males affected by moderate to severe oligo-astheno-teratospermia**

| | Myoinositol + melatonin | |
|---|---|---|
| | N=10 | |
| | Before treatment | After treatment |
| FSH | 8.2 | 6.5 |
| Testosterone | 0.45 | 0.75 |
| Average concentration of spermatozoa (10 x 6 /ml) | 7 ± 4 | 10 ± 5 |
| % progressive straight motility | 8 | 14 |
| % non progressive straight motility | 22 | 27 |
| % normal shapes | 11 | 16 |

The combined treatment of melatonin and myoinositol is thus capable of improving significantly the peripheral activity of FSH and LH hormones, by implementing and regularizing both female and male gametogenesis.

Moreover, the folic acid is able to improve this synergism by acting as a further regulator of cell growth (in this case folliculogenesis and spermatogenesis).

## Claims

1. A composition consisting of 1000 to 4000 parts by weight of myo-inositol and 1 to 5 parts by weight of melatonin and optionally 0.2 to 0.8 parts by weight of folic acid.

2. The composition according to claim 1 for use as a food supplement.

3. The composition according to claim 1 for use as a pharmaceutical drug for administration to a female or male subject.

4. The composition according to claim 3, wherein said administration is in a daily quantity that comprises 1 to 4 g of myoinositol, 1 to 5 mg of melatonin and, if folic acid is present, 200 to 800 µg of folic acid.

5. The composition according to claim 4, wherein said subject is selected from the group constituted by a woman with menstrual cycle disorders, a woman affected by micropolycystic ovary syndrome, a woman affected by genito-pelvic endometriosis, a woman affected by adolescent age ovulation disorders, a woman affected by perimenopausal age ovulation disorders, a woman affected by fertility reduction due to advanced biological age (> 35 years), and a woman affected by fertility reduction due to a reduced ovarian reserve (FSH > 10 mUI/ml).

6. The composition according to claim 4, wherein said subject is a woman subjected to induction of ovulation for medically assisted procreation.

7. The composition according to claim 4, wherein said subject is affected by spermatogenesis disorders as a consequence of imbalances and/or altered rhythms of production of FSH and LH hormones.

8. A unit dosage form of a composition according to one of the preceding claims, comprising 1 to 4 g of myoinositol, 1 to 5 mg of melatonin and 200 to 800 µg of folic acid.

9. The dosage form according to claim 8, comprising 2 g of myoinositol, 3 mg of melatonin and 400 µg of folic acid.

10. Use of a composition according to claim 1 for preparation of a pharmaceutical drug for the treatment of micropolycystic ovary, genito-pelvic endometriosis, ovulation disorders.

## Patentansprüche

1. Mischung bestehend aus 1.000 bis 4.000 Gewichtsanteilen Myo-Inositol und 1 bis 5 Gewichtsanteilen Melatonin und optional 0,2 bis 0,8 Gewichtsanteilen Folsäure.

2. Mischung gemäß Anspruch 1 zur Verwendung als Nahrungsergänzungsmittel.

3. Mischung gemäß Anspruch 1 zur Verwendung als pharmazeutisches Arzneimittel zur Verabreichung an weibliche oder männliche Personen.

4. Mischung gemäß Anspruch 3, wobei die Verabreichung eine tägliche Menge von 1 bis 4 g Myo-Inositoi, 1 bis 5 mg Melatonin und, bei Anwesenheit von Folsäure, 200 bis 800 µg Folsäure.

5. Mischung gemäß Anspruch 4, wobei die Person aus einer Gruppe ausgewählt wird, die aus einer Frau mit Monatsbeschwerden, einer Frau mit mikropolyzystischem Ovarialsyndrom, einer Frau mit Genital-Becken-Endometriose, einer Frau mit Ovulationsstörungen in der Jugend, einer Frau mit Ovulationsstörungen in den Wechseljahren, einer Frau mit Fertilitätsreduktion aufgrund von fortgeschrittenem biologischem Alter (> 35 Jahre) und einer Frau mit Fertilitätsreduktion aufgrund von reduzierter ovarieller Reserve (FSH > 10 mUl/ml) gebildet wird.

6. Mischung gemäß Anspruch 4, wobei die Person eine Frau ist, bei der zur medizinisch unterstützten Zeugung eine Ovulationsinduktion durchgeführt wird.

7. Mischung gemäß Anspruch 4, wobei die Person infolge von Ungleichgewicht und / oder verändertem Produktionsrhythmus von FSH und LH Hormonen von Störungen der Spermatogenese beeinträchtigt ist.

8. Dosierungseinheit einer Mischung gemäß einem der vorigen Ansprüche, mit 1 bis 4 g Myo-Inositol, 1 bis 5 mg Melatonin und 200 bis 800 µg Folsäure.

9. Darreichungsform gemäß Anspruch 8, mit 2 g Myo-Inositol, 3 mg Melatonin und 400 µg Folsäure.

10. Verwendung einer Mischung gemäß Anspruch 1 zur Herstellung eines pharmazeutischen Arzneimittels zur Behandlung von mikropolyzystischem Ovarialsyndrom, Genital-Becken-Endometriose, Ovulationsstörungen.

## Revendications

1. Composition constituée de 1000 à 4000 parties en poids de myo-inositol et de 1 à 5 parties en poids de mélatonine et éventuellement de 0,2 à 0,8 partie en poids d'acide folique.

2. Composition selon la revendication 1 pour une utilisation en tant que complément alimentaire.

3. Composition selon la revendication 1 pour une utilisation en tant que médicament pharmaceutique pour une administration à un sujet de sexe féminin ou masculin.

4. Composition selon la revendication 3, dans laquelle ladite administration est dans une quantité journalière qui comprend 1 à 4 g de myo-inositol, 1 à 5 mg de mélatonine et, si de l'acide folique est présent, 200 à 800 µg d'acide folique.

5. Composition selon la revendication 4, ledit sujet étant choisi dans le groupe constitué d'une femme souffrant de troubles du cycle menstruel, d'une femme touchée par le syndrome des ovaires micropolykystiques, d'une femme touchée par une endométriose génito-pelvienne, d'une femme touchée par des troubles de l'ovulation à l'adolescence, d'une femme touchée par des troubles de l'ovulation à la périménopause, d'une femme touchée par une diminution de la fertilité due à un âge biologique avancé (> 35 ans), et d'une femme touchée par une réduction de la fertilité due à une réduction de la réserve ovarienne (FSH > 10 mUI/ml).

6. Composition selon la revendication 4, dans laquelle ledit sujet est une femme soumise à une induction de l'ovulation pour une procréation médicalement assistée.

7. Composition selon la revendication 4, dans laquelle ledit sujet est touché par des troubles de la spermatogenèse comme conséquence de déséquilibres et/ou de rythmes modifiés de la production des hormones FSH et LH.

8. Forme galénique unitaire d'une composition selon l'une des revendications précédentes, comprenant 1 à 4 g de myo-inositol, 1 à 5 mg de mélatonine et 200 à 800 µg d'acide folique.

9. Forme galénique selon la revendication 8, comprenant 2 g de myo-inositol, 3 mg de mélatonine et 400 µg d'acide folique.

10. Utilisation d'une composition selon la revendication 1 pour la préparation d'un médicament pharmaceutique destiné au traitement des ovaires micropolykystiques, de l'endométriose génito-pelvienne, des troubles de l'ovulation.
